Europäisches Patentamt

European Patent Office

Office européen des brevets

(11) Publication number: **0 235 928 B1**

## EUROPEAN PATENT SPECIFICATION

(45) Date of publication of patent specification: **17.04.91**  (51) Int. Cl.⁵: **A61F 5/441**

(21) Application number: **87300747.0**

(22) Date of filing: **28.01.87**

(54) Deodorizing filters for ostomy equipment.

(30) Priority: **31.01.86 DK 481/86**

(43) Date of publication of application:
**09.09.87 Bulletin 87/37**

(45) Publication of the grant of the patent:
**17.04.91 Bulletin 91/16**

(84) Designated Contracting States:
**DE ES FR GB IT**

(56) References cited:
**EP-A- 0 089 110**    **EP-A- 0 092 299**
**GB-A- 1 363 644**    **GB-A- 1 550 960**
**GB-A- 1 571 382**    **US-A- 3 759 260**
**US-A- 4 274 848**

(73) Proprietor: **COLOPLAST A/S**
**4, Bronzevej**
**DK-3060 Espergaerde(DK)**

(72) Inventor: **Ballan, Akeel**
**Pragtsjernevej 1**
**DK-2400 Copenhagen NV(DK)**

(74) Representative: **Davies, Christopher Robert et al**
**Frank B. Dehn & Co. Imperial House 15-19 Kingsway**
**London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to deodorizing filters for ostomy equipment, in particular ostomy bags, of the type wherein the active filter material is a substantially flat, porous, elongate filter body of a material such as activated carbon textile material or foamed plastics impregnated with highly activated carbon.

Several filters are known for use in various forms of ostomy equipment, most frequently ostomy bags. Ostomies are surgically made intestinal openings in the abdominal wall for the discharge of visceral contents in cases where for some reason it has been necessary to discontinue the natural discharge of fecal matter. The discharge of visceral contents including intestinal gases cannot be regulated at will, and for that purpose the intestinal opening may be closed with a closure means, e.g. a tampon or a magnetic closure, or the contents may be collected in a bag which is later emptied and/or discarded at suitable times. The discharge of flatus, measured in volume, may exceed the discharge of solid and liquid fecal matter by many hundred percent and therefore there is usually the need for the continuous or frequent venting of the intestine or of the collecting bag. Normally the outflowing flatus is dodorized with a suitable filter. Commonly the active filter material is powdered active carbon, which absorbs $H_2S$ being the principal component of flatus.

Examples of such filters are described in US patent specifications Nos. 3,804,091, 3,952,727 and 3,759,260 wherein activated carbon particles are present in a filter body of matted fibers and wherein the filter body is arranged such that the flatus passes therethrough in various ways. However, it is a drawback of the filters known from these specifications that there is no firm surface connection between the filter body and the plastic, gas impermeable cover sheets therefor whereby a flow of gases may occur through the filter without passing through the filter body and hence without being deodorized. This for instance may take place as a result of bending or bowing of the filter consequent e.g. upon the patient bending, which can cause small interspaces or channels to be formed between the filter body and one of the walls.

US patent specification No. 4,490,145 discloses an ostomy pouch with a deodorizing filter. The filter consists of a filter element affixed to the outside or inside of the pouch outer wall and comprising a polymeric film cover attached to the said pouch wall, and an insert of a gas deodorizing material, e.g. of one of the kinds mentioned above. The pouch wall has an aperture providing entry to the filter element and the polymeric film cover has an aperture for venting the deodorized gas to the atmosphere. The two apertures are spaced from each other in order to let the intestinal gas pass through the length of the insert of deodorizing material. US specification No. 4,490,145 does not disclose a positive sealing connection between the film cover for the filter element and the insert of deodorizing material but in a commercial ostomy pouch, "ConvaTex" made in accordance with this US patent one surface of the deodorizing material is sealed to the outer pouch wall whereas the opposite surface of the dodorizing material is not sealed to the polymeric film cover; accordingly there is a space between the deodorizing material and the cover through which gas can flow freely, especially in cases of bending of the filter. This means that the intestinal gas can pass directly across the thickness of the deodorizing material, i.e. about I or 2 mm, and then through the space defined between such material and the polymeric film cover. Satisfactory deodorizing cannot therefore be achieved.

A similar filter construction is disclosed in DK patent application No. 3616/76 in which the filter element comprises a filter housing with apertures for the inlet and outlet of gases and containing an odour absorbing material. The latter may be connected with one side of the filter housing and the two apertures are spaced as far from each other as possible. Again, since only one side of the absorbing material is connected to a wall of the housing, the gas may pass between that wall and the absorbing material and may thus only be subjected to deodorizing as a result of passing directly through the thickness of the absorbing material.

These drawbacks have been overcome by the filters described in GB 1,571,382 and EP 0089,110 in the name of Coloplast A/S, wherein a plastic wall has been glued or hot sealed to a filter body consisting of an open cell foam plastic impregnated with the activated carbon particles. These filters preferably are formed as circular discs of a thickness of 2-3 mm and a diameter of 25-30 mm and having an aperture in the centre of the filter material and in one of the walls arranged such that the flatus enters through the aperture in the wall and flows into the filter material through the edges of the aperture therein, and flows radially through the filter and leaves it through its cylindrical outer edge.

These filters function well and are particularly suitable for ostomy bags. They are adapted to yield a small gas resistance within the filter material and hence a low drop of pressure, and the same is true for the arrangements known from the US specifications discussed above. The purpose of the low drop of pressure is to ensure that the bag does not inflate and thereby become visible on the outside of the wearer's clothing, which should be avoided for obvious reasons.

If, however, the drop of pressure becomes too low this can also create problems. Firstly it can occur that the entire volume of gas in the bag escapes so that the walls of the bag, consisting of a smooth plastic sheet material, stick together and also stick to the ostomy. This prevents faeces from the ostomy from falling to the bottom of the bag, and may also increase the risk of clogging the venting hole or holes in the wall of the bag. Sooner or later this can result in unacceptable bulging of the bag.

Secondly, a too high flow velocity through the filter may result in inefficient deodorizing of the intestinal gases flowing out through the filter.

The problem of avoiding a sudden fall of gas pressure in the interior of the pouch has also been taken into account in the above-mentioned US Patent No. 4,490,145 in which it is proposed, in order to keep the gas deodorizing material dry and control the rate at which gas can pass from the pouch into the filtering element, to cover the inlet and/or outlet aperture by a barrier layer of a gas permeable, liquid impermeable material. However, such barrier layers will not only create a certain distension of the pouch itself, but also tend to cause distension between the insert of gas deodorizing material and the surface thereof not sealed to the pouch wall and hence promote the above-mentioned tendency for the gas to travel through only the thickness of the insert and not along its length. This in turn will promote a fall of gas pressure in the interior of the pouch.

Thus, there is a need to avoid the drawbacks of known arrangements mentioned above and to provide venting for ostomy bags and the like wherein the drop of pressure is higher than in known filters so that the bag is maintained inflated to a suitable degree by the intestinal gas which flows in more or less continuously. It will be understood that if the wall of the bag opposite the filter sticks to the opposite wall a sufficiently strong build up of pressure will normally eventually release the "sticking" However, such an inflated state will be unmistakable on the outside of the patient's clothing, which for psychological reasons clearly should be avoided. On the other hand, the pressure drop through the filter should not be so big such that high inflation of the bag occurs in any case.

Consequently it is one object of the invention to provide a filter for ostomy bags and the like in which a higher drop of pressure occurs therein than in known filters, but wherein the drop of pressure is not so great as to cause over inflation of the bag.

Viewed from one aspect the invention provides a deodorizing filter for ostomy equipment or the like, the filter comprising an elongate, substantially flat filter body of porous filter material interposed between gas- and liquid-impervious walls which are hermetically sealed to the body along its longitudinal side edges, gas inlet and outlet openings being provided in communication with the filter material adjacent its respective longitudinal end regions characterized in that both of the gas-and liquid-impervious walls are hermetically sealed to the upper and lower surfaces of the filter body the arrangement being such that in use gas flows longitudinally through said filter from the inlet opening to the outlet opening, such gas flow being confined to said filter element.

Preferably the thickness of the filter body is 0.25 to 3.0 mm and the distance between the inlet opening and the outlet opening, as measured between the edges thereof which are closest together, is at least 10 mm.

The two walls mentioned, which together form the filter housing, may be of any plastic sheet material normally used in connection with ostomy equipment, in particular ostomy bags; the only condition is that the material should be compatible with the plastic material of which the ostomy equipment in question is made, at least in the region of the side thereof to which the filter is located. In the case of ostomy bags the filter is preferably placed on the outside of the bag. When used in connection with tampons as described in e.g. Danish patent application 187/85, see especially figs. 5-7, it may be placed on the side of a cover sheet which faces the body of the wearer or on the side thereof which faces away from the body. When a filter in accordance with the invention is placed on the side of a bag or cover sheet which in use faces away from the body, there are openings in the bag or sheet permitting the intestinal gas flow to the inlet opening of the filter housing, and if the filter is placed on the inner side of the bag or cover sheet, there are preferably openings therein which communicate with the outlet opening of the filter housing. Mounting of an ostomy bag or other ostomy equipment normally takes place by heat sealing, glueing or by other similar means.

The filter body may be formed in various ways and may advantageously consist of a compressed foamed plastic impregnated with (i.e. containing in its pores) a highly activated carbon. This may for instance be activated by a metal salt, notably a heavy metal salt. A suitable material of this kind is known under the trade name "LR96 Charcoal impregnated foam", supplied by Bondina Industrial, Greetland, Halifax, England. In another embodiment the filter body consists of an activated carbon textile material. Such carbon textile material is known from e.g. the paper by Richard B. Macnair and Gilbert Arons, "Absorptive Textile Systems Containing Activated Carbon Fibres" in P.N Cherimisonoff and F. Elabsh, "Carbon Absorption Handbook" (1978), Chapter 22.

The materials are known commercially under various names, e.g. "Charcoal Cloth", "C-Tec activated Carbon Textile" and "RK Carbon fibres". They are made from fibers, yarn or woven or knitting textile articles by the pyrolysis into carbon in an inert atmosphere at a temperature up to 500°C, followed by activation (formation of pores and surface area) at a higher temperature, about 1000°C, in an oxidating atmosphere, e.g. carbon dioxide or superheated steam. Such materials possess a high surface area, of the order 250-1200 m²/g because of the formation of small pores. The fibers, yarns or textiles subjected to the pyrolysis according to current information should be of a polymer which carbonizes without melting.

In a leaflet from Siebe Gorman, "C-Tex Activated Carbon Textile" it is stated that such materials have been used for filters for colostomy bags but details are not given.

The entire surface area of the filter material is preferably sealed to the walls e.g. by gluing, heat sealing or other suitable means and the same is true for its edges except adjacent the longitudinal ends of the filter body. The sealing may cause difficulties in some cases but e.g. carbon textile material may be surface fixed by a so-called hot-melt film of a non-woven textile of, e.g., nylon which may thereafter be easily hot-welded to the plastic walls. A suitable material is "Wonder-Web" from Bondina, Great Britain.

The regions of the filter body for the inlet of intestinal gas and outlet of deodorized intestinal gas are in practice most conveniently the longitudinal end edges of the filter body but may alternatively be e.g. surface area regions closely bordering the end edges.

As mentioned above the filter should provide a drop of pressure which is bigger than the drop of pressure in known filters but not so high as to not cause undesirably high inflation of the bag. By suitable relative dimensioning of the thickness and possible width of the filter and the distance between the openings it is expedient in practice that the pressure drop through the filter corresponds to at least 70 and at most 175 mm water column at a flow velocity of 50 ml gas or gas mixture per minute when the filter body is of the kind comprising compressed foam plastic imgregnated with carbon highly activated by heavy metal salts. In the case where the filter body is formed of a carbon textile material the drop of pressure is typically somewhat higher, e.g. 75-250 mm water column at a flow of the gas or gas mixture of 25 m l/minute.

The arrangement in practice of the filter body and the walls may vary. In one advantageous embodiment the inlet opening for intestinal gas and the outlet opening for deodorized intestinal gas are situated in the walls adjacent or directly outside and opposite the end edges of the filter body, the length of the filter body being at least 15 mm, preferably at least 20 mm and expediently 25-35 mm. The intestinal gas thereby enters the filter through one end edge and after deodorizing escapes through the other, and if the two openings are substantially identical and extend substantially across the entire end edges of the filter body, it is then the width and the thickness of the openings that determine the active cross sectional area of the filter and thereby the openings are an important factor for determining the pressure drop through the filter. With a width of the filter body of at least about 10 mm, a length of 30 mm and a thickness of about 1.5 mm in the case of impregnated foam plastic and about 0.5 mm in the case of a carbon textile material there is, in a preferred embodiment, obtained a drop of pressure in the above-mentioned desirable ranges.

The inlet opening defined by the filter walls for intestinal gas and the outlet opening for deodorized intestinal gas as mentioned conveniently have the same length as the width of the filter body. The width of these openings - i.e. the dimension in the longitudinal direction of the filter body -may be nil, that is they can be slits cut in the walls, but alternatively they may advantageously be 0.5-1 mm wide.

The preferred filter so far described can be used for colostomy bags and other equipment for colostomy but is less suitable for ileostomy equipment. The reason for this is that the fecal matter discharged from ileostomies has a rather thin liquid form yet also contains solid particles. It has been found that this may lead firstly to the inlet opening for intestinal gas being substantially blocked by solid particles carried by outflowing liquid, and secondly to liquid penetrating into the filter body and entirely or partly blocking it.

It has been found that this drawback is avoided in a preferred embodiment wherein the filter wall adapted to face the source of intestinal gas is covered by a layer of sheet material, preferably plastic sheet material connected to the filter walls and, inside the periphery of this connection, provided with openings for the passage of intestinal gas. Conveniently these openings, which may for instance be slits of a width of 0-0.5 mm and a length of a few millimeters, may be situated outside the peripheral edge of the filter body.

Another possibility of avoiding or reducing the drawback described above, which may render a preferred filter suitable for use with ileostomy equipment, involves providing the filter housing on the surface adapted to face the source of intestinal gas with a layer of liquid-absorbing material. This material may for instance be filter paper, cellulose fluff, other absorbent cellulosic material, or be of a highly absorbent kind prepared from powdery or fibrous materials on the basis of cellulose or starch products, cross-linked acrylic or methacrylic polymers or combinations thereof, e.g. those known under the trade name "Favor" and supplied by Chemische Fabrik Stockhausen, Krefeld, BRD. The area of the absorbent material is conveniently the same as the area of the filter body or somewhat larger but the positioning on the wall in question should be such that the absorbent material does not cover the inlet opening for intestinal gas to be deodorized since saturation of the liquid-absorbing material with liquid might prevent the passage of the intestinal gas. The thickness of the absorbent layer of material may vary within wide limits, depending on its absorbancy, its area, the precise positioning of the filter in relation to the ostomy equipment in question, e.g. an ostomy bag, and possibly the expected flow of liquid material. This may vary from patient to patient, and there may be manufactured filters adapted to absorb bigger or lesser amounts of liquid.

It is also possible to render the filter particularly efficient for ileostomy equipment by providing it both with a covering sheet and an absorbent layer of material. Thus, in a further preferred embodiment an absorbent layer of material is secured to the covering layer of plastic sheet, preferably on the side thereof facing away from the filter. In this arrangement the absorbent layer of material should be placed on the covering plastic sheet so that it does not significantly cover the openings for passage of inflowing intestinal gas. In practice this may conveniently be achieved by forming these openings as apertures of arbitrary shape, e.g. circular, or slits formed by simply cutting the sheet without removing any material, the apertures or slits being positioned in a suitable number at a suitable mutual distance adjacent, but outside the absorbent material.

Certain embodiments of the invention will now be described, by way of example only, with reference to the drawings, wherein:

Fig. 1 shows an embodiment of filter in accordance with the invention, which is particularly suitable for colostomy equipment, in plan view;

Fig. 2 is a longitudinal section of the same filter along the line II-II in Fig. 1;

Fig. 3 is is a cross-section of the embodiment of Fig. 1 along the line III-III in Fig. 1;

Figs. 4-6 are longitudinal sections, analogous to Fig. 2, of three other embodiments of filter in accordance with the invention, suitable for ileostomy equipment; and

Figs. 7-8 are curves showing the pressure drop through various filters in accordance with the invention and, for comparison, through a known filter commercially available under the name "Filtrodor"[R].

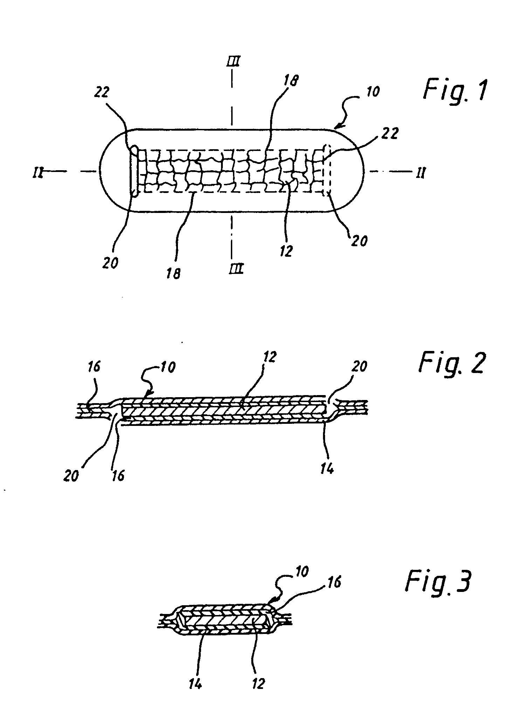

The thickness dimensions of the layers of plastic sheets and adhesives are much exaggerated compared to the length and width dimensions in Figs. 1-6; the latter and the thickness of the filter bodies are not necessarily shown in their actual sizes, yet are shown in the correct order of magnitude.

A filter 10 shown in Figs. 1-3 has a filter body 12, preferably of one of the two types described hereinbefore. The filter body 12 is tightly encased by two plastic sheet walls 14. Plastic sheet walls 14 together form a filter housing and are firmly secured to the entire main surfaces of the filter body 12 by means of layers 16 of adhesive or hot melt material. The plastic sheet walls 14 extend beyond the outer periphery of filter body 12 and are joined together by a layer 16 of adhesive or hot melt material. They may also be welded to each other without any separate bonding agent. If, for instance because of the thickness of the sheet material, there may be a danger that an interspace is formed between the longitudinal edges (side edges) 18 of the filter body and the joint between the two walls opposite these edges, the longitudinal edges of the filter body may be sealed to the walls preferably means of the same adhesive or hot melt material. Each of the walls 14 has one opening 20, the opening 20 in one wall being directly opposite one end edge 22 of the filter body and the opening in the other end edge being directly opposite the other end edge 22 of the filter body. In the embodiment shown, the openings 20 are elongated, having about the same length as the width of the filter body. The width of the elongated openings 20 (i.e. the dimension in the longitudinal direction of the filter body) is shown as being about the same as the thickness of the filter body but may in practice merely be slits having a width of 0 mm (no sheet material punched away); they may also be wider than shown or be slits or round apertures located opposite the surfaces of the filter body.

The filter housing, i.e. the walls 14, are formed of a sheet material which must be compatible with the sheet material of which the ostomy equipment, e.g. a colostomy bag or an ileostomy bag, is made. It has been found practical that both the bag and the filter housing consist of a plastic laminate consisting of two outer layers of EVA-sheet (EVA: ethylene-vinyl acetate copolymer) with an intermediate layer of PVDC (polyvinylidene chloride). Such a laminate imparts a certain rigidity which may be desirable, especially in connection with the embodiments described below.

The filter 10 is secured in a suitable manner to an ostomy bag or other ostomy equipment, e.g. by hot melting or sealing. At the location in question on the bag or other ostomy equipment one or more openings for the outflow of intestinal gas are provided, in such a manner that they communicate with the opening 20 in the wall facing the ostomy bag if the filter is placed in the outside of the bag, which is generally preferred. If the filter is placed on the side of the ostomy equipment which faces towards the ostomy opening, e.g. on the inner side of a bag, it is the opening 20 intended for the outlet of deodorized intestinal gas which communicates with an opening in the wall of the ostomy equipment.

The shape of the filter housing may be arbitrarily chosen, but, as shown in Fig. I it is frequently oval or has longitudinal parallel sides and semicircular ends. Suitable dimensions are a length of 50-70, preferably 50-60 mm and a width of 30-50 mm. The filter body will normally be rectangular.

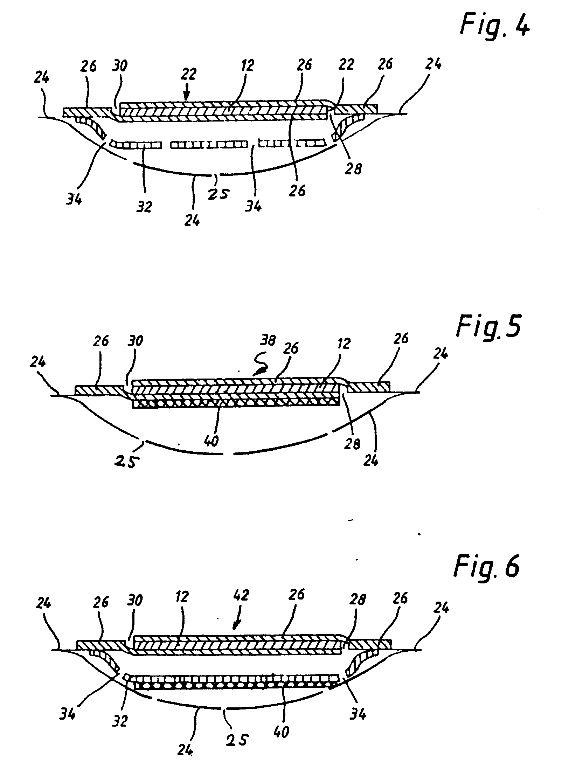

The filters shown in longitudinal section in Figs. 4-6 like that shown in Figs. I-3 have a filter body l2 which is preferably made of one of the materials described hereinbefore, i.e. either a compressed foam material impregnated with highly activated carbon or a carbon textile material. In these figures there is shown a bag wall; the fact that this is merely shown by a thin line does not intend to illustrate any definite thickness relationship between the bag wall and the walls of the filter housing, and the bag may be formed of a thicker or thinner sheet material than the filter housing. In Figs. 4-6 the lower side (hereinafter called the inner side) is intended to face the interior of the bag and hence the source of intestinal gas. In bag wall 24 there are openings 25 for intestinal gas.

For the sake of clarity, filter housing walls (4l in Figs. 2-3) and the material (l6 in Figs. 2-3) by which they have been connected to the filter body l2 are shown as one material in Figs. 4-6, i.e. a filter housing identified by the reference numeral 26. It is not, however, the intention to suggest any essential constructional difference in this respect between the filter of Figs I-3 on one hand, and those of Figs. 4-6 on the other.

On the inner side the filter housing 26 has an inlet opening 28 for intestinal gas, defined adjacent one end edge 22 of the filter body, and on the outer side adjacent the other end edge of the filter body an outlet opening 30 for deodorized intestinal gas. This holds true for all of the three embodiments shown in Figs 4-6.

The inner side of a filter 22 in Fig. 4 on all sides outside the outer periphery of filter body l2 is hermetically connected to a cover sheet 32 provided with a plurality of openings 34, e.g. slits or punched holes, communicating with the interior of the bag and consequently permitting intestinal gas admittance from the bag to inlet opening 28 and from there flow through the length of the filter body to outlet opening where it leaves the filter in the deodorized state. The cover sheet to a considerable degree protects the filter against clogging with thin fecal matter, e.g. gruel like discharges from ileostomies, and against liquid penetrating into the filter body and potentially blocking the gas passage therethrough.

If the cover sheet is of a comparatively thick material, this, in combination with the filter being comparatively rigid as mentioned above, will prevent the cover sheet from bearing closely against the inner side of the filter housing and thereby from blocking the openings 34. The comparatively high drop of pressure through the lengths of the filter housing also contributes to maintaining the openings 34 free. Should one wish to use a cover sheet of a very thin sheet material and a less rigid filter, it would be possible to counteract blocking of the gas passage through openings 34 by the aid of lists or bosses or similar distance pieces located on the inner side of the filter housing or on the side surface of the cover sheet 32 facing that inner side. The fact that cover sheet 32 has a bigger area than the area of the filter housing it spans will also counteract the tendency of the cover sheet to adhere to the filter housing and block the openings 34.

The filter 38 shown in Fig. 5 does not have a cover sheet but at the inner side is provided with an adhesively or otherwise secured layer 40 of a liquid-absorbing material. This layer may for instance have the same shape and size as filter body l2, but alternatively it may be larger provided that it does not cover the inlet opening 28 to the filter. The layer 40 counteracts the tendency for large amounts of liquid to penetrate into the filter body and block its pores.

The filter 42 shown in Fig. 6 is a combination of the embodiments shown in Fig. 4 and Fig. 5, and is provided both with a cover sheet 32 having perforations or other openings 34, and with a liquid-absorbing layer 40. In filter 42 the liquid-absorbing layer 40 is shown as positioned on the side of the cover sheet facing the bag and hence facing away from filter housing 26. Such an absorbing material might instead be positioned on the side of the cover sheet 32 facing the inner side of the filter, or there might be absorbing material layers 40 on both sides of cover sheet 32. In any case the openings 34 must not be covered by the absorbing material, on either side.

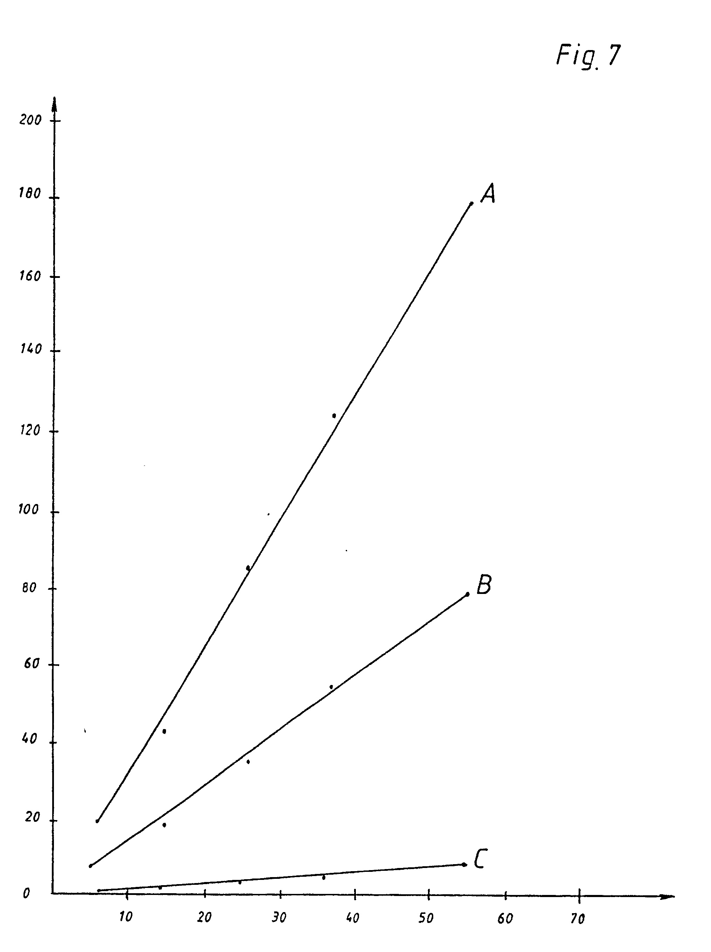

Fig. 7 shows the pressure drop against air flow through preferred filters in accordance with the invention of the type shown in Figs. I-3, other than in that the inlet and outlet openings 20 are merely slits extending along the entire edge of the filter body. The abscissa shows the flow of air in ml/minute whereas the ordinate shows the pressure drop in mm water column. The filter body was of the kind described hereinbefore, "LR96 charcoal impregnated foam" from Bondina International. The length of the filter body was 3 cm, its width I cm and its thickness about I.5 mm.

A number of experiments were carried out with various filters and the results showed some scattering, possibly because there may be small differences in the exact position of the slits constituting the inlet and outlet openings 20. In Fig. 7 curve A shows the upper limit of pressure drop in these experiments and curve B the lower limit. Curve C is a curve of the corresponding drop of pressure through a commercial filter, "Filtrodor" (RTM).

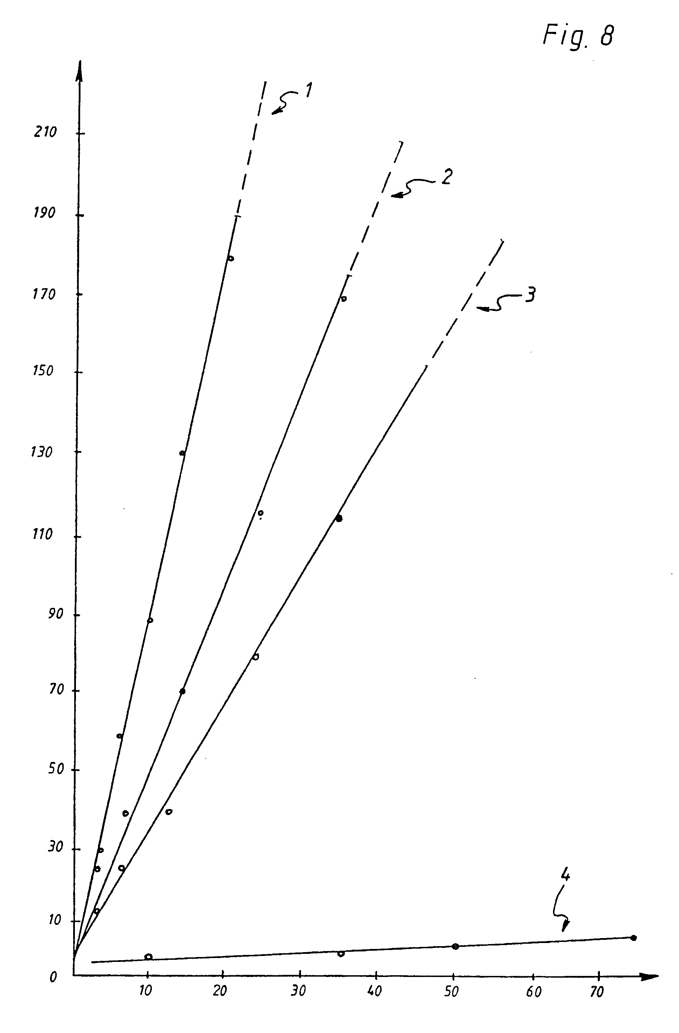

Fig. 8 shows in the same manner the pressure drop through some embodiments of filters in accordance with the invention in which the filter body was of a carbon textile as explained hereinbefore, viz. that denoted "C-Tex" Standard No. 0553010I from Siebe Gorman. Abscissa and ordinate in Fig. 8 are as in fig. 7.

In Fig. 8 curve I shows the pressure drop through a filter constructed as shown in Figs. I-3 having a length of the filter body of 20 mm, width of I5 mm and thickness of 0.5 mm, the inlet and outlet openings being 0.5-I mm wide and extending along the entire end edge of filter body I2.

Curve 2 shows the pressure drop through a similar filter having the same dimensions of the filter body but wherein the ends of the latter were closed and instead the inlet and outlet openings were circular holes of a diameter of 2-2.5 mm at a distance of I-I.5 mm from the ends of the filter body. In this embodiment the filter body flush with the circular inlet and outlet region of the openings in wall I4 is recessed through to the opposite wall I4, the recesses having the same diameter as the inlet/outlet openings so that the admittance to and discharge from the filter body is via the cylindrical surfaces bordering the recesses in the filter material.

Curve 3 shows the pressure drop through a similar filter, but having a hole diameter of 3 mm.

Finally, curve 4 shows the pressure drop through a commercial deodorizing filter "Filtrodor"[R].

## Claims

1. A deodorizing filter for ostomy equipment or the like, the filter comprising an elongate, substantially flat filter body (12) of porous filter material interposed between gas- and liquid-impervious walls (14;26) which are hermetically sealed to the body (12) along its longitudinal side edges, gas inlet and outlet openings (20;28;20;30) being provided in communication with the filter material adjacent its respective longitudinal end regions (22), characterized in that both of the gas- and liquid-impervious walls (I4;26) are hermetically sealed to the upper and lower surfaces of the filter body (12) the arrangement being such that in use gas flows longitudinally through said filter from the inlet opening (20;28) to the outlet opening (20;30), such gas flow being confined to said filter element.

2. A deodorizing filter according to claim 1, characterized in that the thickness of the filter body (12) is 0.25 to 3.0 mm and the distance between the inlet opening (20;28) and the outlet opening, as measured between the edges thereof which are closest together, is at least 10 mm.

3. A deodorizing filter according to claims 1 or 2, characterised in that the thickness of the filter body (12) is 0.5 to 2.5 mm.

4. A deodorizing filter according to claim 1, 2 or 3 characterised in that the distance between the inlet opening (20;28) and the outlet opening (20;30) as measured between the edges thereof which are closest together, is 30 mm.

5. A deodorizing filter according to any preceding claim characterised in that the filter wall having the inlet opening (28) is covered by a plastic cover sheet (32) sealed thereto around its outer periphery, such sheet being provided with openings for the passage of intestinal gas.

6. A deodorizing filter according to any of claims 1 to 4 characterised in that a layer (40) of a liquid-absorbing material is secured to the filter wall having the inlet opening (28), on the side thereof which faces in use a source of intestinal gas.

7. A deodorizing filter according to claim 5, characterised in that a layer (40) of a liquid-absorbing material is secured to the plastic cover (32).

8. A deodorizing filter according to claim 7, characterised in that the layer (40) of liquid-absorbing material is secured to the surface of the cover sheet (32) which faces away from the filter body (12).

9. A deodorizing filter according to any preceding claim characterised in that the inlet opening (20;28) and the outlet opening (20;30) are in the form of substantially identical slits formed in the respective walls and extending substantially across the entire end edges (22) of the filter body.

10. A deodorizing filter as claimed in any preceding claim wherein the filter material of the filter body comprises either activated carbon textile material or foamed plastics impregnated with activated carbon.

## Revendications

1. Filtre désodorisant pour équipement d'ostomie ou équivalent, le filtre comprenant un corps de filtre allongé sensiblement plat (12) formé d'un matériau filtrant poreux interposé entre des parois étanches aux liquides et aux gaz (14; 26) qui sont hermétiquement scellées au corps (12) le long de ses bords latéraux longitudinaux, des ouvertures d'entrée et de sortie de gaz (20; 28; 20; 30) étant disposées en communication avec le matériau filtrant au voisinage de ses zones (22) d'extrémités longitudinales respectives, caractérisé en ce que les deux parois imperméables aux liquides et aux gaz (14,26) sont scellées hermétiquement aux surfaces supérieures et inférieures du corps de filtre (12), la disposition étant telle qu'en utilisation, le gaz s'écoule longitudinalement à travers ledit filtre depuis l'ouverture d'entrée (20,28) vers l'ouverture de sortie (20,30), un tel écoulement de gaz étant confiné audit élément de filtre.

2. Filtre désodorisant selon la revendication 1, caractérisé en ce que l'épaisseur du corps de filtre (12) est 0,5 à 3 mm, et la distance entre l'ouverture d'entrée (20,28) et l'ouverture de sortie, telle que mesurée entre leurs bords les plus proches, est au moins 10 mm.

3. Filtre désodorisant selon la revendication 1 ou la revendication 2, caractérisé en ce que l'épaisseur du corps de filtre (12) est 0,5 à 2,5 mm

4. Filtre désodorisant selon la revendication 1, la revendication 2 ou la revendication 3, caractérisé en ce que la distance entre l'ouverture d'entrée (20,28) et l'ouverture de sortie (20, 30), telle que mesurée entre leurs bords les plus proches, est 30 mm.

5. Filtre désodorisant selon l'une quelconque des revendications précédentes, caractérisé en ce que la paroi de filtre ayant l'ouverture d'entrée (28) est couverte par une feuille de couverture de plastique (32) scellée à elle autour de son bord extérieur, une telle feuille étant munie d'ouvertures pour le passage de gaz intestinaux.

6. Filtre désodorisant selon l'une quelconque des revendications 1 à 4, caractérisé en ce qu'une couche (40) de matériau absorbant les liquides est fixée à la paroi de filtre ayant l'ouverture d'entrée (28) sur son côté qui fait face en utilisation à une source de gaz intestinal.

7. Filtre désodorisant selon la revendication 5, caractérisé en ce qu'une couche (40) de matériau absorbant de liquides est fixée à la couverture de plastique (32).

8. Filtre désodorisant selon la revendication 7, caractérisé en ce que la couche (40) de matériau absorbant de liquides est fixée à la surface de la feuille de couverture (32) qui ne fait pas face au corps de filtre (12).

9. Filtre désodorisant selon l'une quelconque des revendications précédentes, caractérisé en ce que l'ouverture d'entrée (20,28) et l'ouverture de sortie (20,30) ont la forme de fentes sensiblement identiques formées sur les parois respectives et s'étendant sensiblement sur toute la longueur des bords d'extrémités (22) du corps de filtre.

10. Filtre désodorisant selon l'une quelconque des revendications précédentes, caractérisé en ce que le matériau filtrant du corps de filtre comprend soit un matériau textile à carbone activé, soit des mousses plastiques imprégnées de carbone activé.

## Ansprüche

1. Geruchsfilter für Colostomiebeutel od. dgl., welcher einen länglichen, im wesentlichen flachen Filterkörper (12) aus porösem Filtermaterial aufweist und zwischen gas- und flüssigkeitsundurchlässigen Wänden (14; 26) angesetzt ist, welche zu dem Filterkörper (12) entlang ihrer Längsseitenkanten hermetisch abgedichtet sind, wobei Gaseinlaß- und -auslaßöffnungen (20; 28; 20; 30) in Verbindung mit dem Filtermaterial benachbart zu dessen entsprechenden Längsendbereichen (22) vorgesehen sind, dadurch gekennzeichnet, daß beide gas- und flüssigkeitsundurchlässige Wände (14; 26) hermetisch zu der oberen und unteren Oberfläche des Filterkörpers (12) abgedichtet sind, und daß die Anordnung so getroffen ist, daß im Gebrauch das Gas längs durch den besagten Filter von der Einlaßöffnung (20; 28) zu der Auslaßöffnung (20; 30) strömt und dieses Gas nur durch das Filterelement strömen kann.

2. Geruchsfilter nach Anspruch 1, dadurch gekennzeichnet, daß die Dicke des Filterkörpers (12) 0,25 bis 3,0 mm beträgt und der Abstand zwischen der Einlaßöffnung (20; 28) und der Auslaßöffnung, gemessen zwischen den am nächsten zueinander liegenden Rändern dieser Öffnungen, mindestens 10 mm groß ist.

3. Geruchsfilter nach Anspruch 1 oder 2, dadurch gekennzeichnet, daß die Dicke des Filterkörpers (12) 0,5 bis 2,5 mm beträgt.

4. Geruchsfilter nach Anspruch 1, 2 oder 3, dadurch gekennzeichnet, daß der Abstand zwischen der Einlaßöffnung (20; 28) und der Auslaßöffnung (20,; 30), gemessen zwischen den am nächsten zueinander liegenden Rändern dieser Öffnungen, 30 mm beträgt.

5. Geruchsfilter nach einem der vorhergehenden Ansprüche, dadurch gekennzeichnet, daß die Filterwand, die eine Einlaßöffnung (28) aufweist, durch eine Plastikabdeck-folie (32) abgedeckt und hierzu auf dem äußeren Umfang abgedichtet ist und daß diese Folie mit Öffnungen für den Durchgang von Darmgasen versehen ist.

6. Geruchsfilter nach einem der Ansprüche 1 bis 4, dadurch gekennzeichnet, daß eine Schicht (40) eines flüssigkeitsabsorbierenden Materials fest an der Filterwand mit der Einlaßöffnung (28) an derjenigen Seite aufgebracht ist, die im Gebrauch gegenüber der Quelle der Darmgase liegt.

7. Geruchsfilter nach Anspruch 5, dadurch gekennzeichnet, daß eine Schicht (40) eines flüssigkeitsabsorbierenden Materials an der Plastikabdeckung (32) befestigt ist.

8. Geruchsfilter nach Anspruch 7, dadurch gekennzeichnet, daß die Schicht (40) aus flüssigkeitsabsorbierendem Material auf derjenigen Oberfläche der Abdeckfolie (32) befestigt ist, welche auf der dem Filterkörper (12) abgewandten Seite liegt.

9. Geruchsfilter nach einem der vorhergehenden Ansprüche, dadurch gekennzeichnet, daß die Einlaßöffnung (20; 28) und die Auslaßöffnung (20; 30) in Form von im wesentlichen identischen Schlitzen in den jeweiligen Wänden ausgebildet sind und sich im wesentlichen über die gesamten Endkanten (22) des Filterkörpers erstrecken.

10. Geruchsfilter nach einem der vorhergehenden Ansprüche, dadurch gekennzeichnet, daß das Filtermaterial des Filterkörpers entweder Aktivkohle-Textilmaterial oder mit Aktivkohle imprägnierten Schaumkunststoff aufweist.

Fig. 1

Fig. 2

Fig. 3

Fig. 4

Fig. 5

Fig. 6

Fig. 7

Fig. 8